# EUROPEAN PATENT APPLICATION

(11) **EP 2 486 791 A2**
(43) Date of publication of application: **15.08.2012**
(21) Application number: 10822229.0
(22) Date of filing: 06.10.2010
(51) Int. Cl.: A01H 1/00, C12N 15/40, C12N 15/82, A61K 39/12

(54) **HIGHLY PATHOGENIC AVIAN INFLUENZA VIRUS PROTEIN VACCINE DERIVED FROM TRANSGENIC PLANTS, AND METHOD FOR PREPARING SAME**

(30) Priority: 06.10.2009 KR 20090094627; 04.10.2010 KR 20100096488
(71) Applicant: Helix, Co., Limited, Kyeongsangbuk-do 790-784 (KR)
(72) Inventor: HWANG, In Hwan, Gyeongsangbuk-do Pohang 790-751 (KR); SOHN, Eun Ju, Gyeongsangbuk-do Pohang 790-751 (KR); KWON, Eun Hye, Gyeongsangbuk-do Pohang 790-390 (KR); NA, Yun Jeong, Gyeongsangbuk-do Pohang 790-751 (KR); JEON, Eun Hyun, Gyeongsangbuk-do Pohang 790-390 (KR); IM, Se Jin, Gyeongsangbuk-do Pohang 790-390 (KR); PARK, Ki Seok, Gyeongsangbuk-do Pohang 790-390 (KR); SUNG, Young Chul, Seoul 140-025 (KR)
(74) Representative: Leathley, Anna Elisabeth
(86) International application number: PCT/KR2010/006821
(87) International publication number: WO 2011/043584

(57) **Abstract**

The present invention relates to a method for producing transgenic plants, which involves producing hemagglutinin proteins of the H5N1 virus in a highly efficient manner using a plant transformation recombinant vector, wherein said vector can express, in a highly efficient manner, hemagglutinin proteins of the H5N1 virus which is the highly pathogenic avian influenza virus, and can transport proteins expressed in plants to the endoplasmic reticulum and enable the retention of the proteins in the endoplasmic reticulum so as to accomplish glycosylation which is necessarily required for antigen activity. The present invention also relates to a method for the mass production of hemagglutinin proteins of the antigenic H5N1 virus from the transgenic plants, and to a vaccine composition for the avian influenza virus comprising the transgenic plants or the hemagglutinin proteins produced from the plants. The method for producing hemagglutinin proteins of the H5N1 virus using transgenic plants according to the present invention enables the mass production of hemagglutinin proteins at a low cost. The plant transformation recombinant vector used in the present invention has a cellulose-binding domain inserted therein to separate proteins expressed in plants in a quick and easy manner. In addition, transgenic plants according to the present invention can be taken in the form of feed additives or the like, and therefore can be used as edible vaccines. Further, antigenic hemagglutinin proteins produced by the method induce immune response when administered to an animal model, and therefore can be used not only as protein vaccines for the H5N1 avian influenza virus, but also as diagnostic reagents for avian influenza virus infection.

5. Sequence Listing.txt
<110>    Helix.co.ltd.

<120>    Protein vaccine of high pathogenic avian influenza virus derived
         form transgenic plant and method for preparation thereof

<150>    KR 10-2009-0094627
<151>    2009-10-06

<150>    KR10-2010-0096488
<151>    2010-10-04

<160>    15

<170>    KopatentIn 1.71

<210>    1
<211>    21
<212>    DNA
<213>    Artificial Sequence

<220>
<223>    utr 1


<400>    1
agagaagacg aaacacaaaa g                                                  21


<210>    2
<211>    21
<212>    DNA
<213>    Artificial Sequence

<220>
<223>    utr 2


<400>    2
gagagaagaa agaagaagac g                                                  21


<210>    3
<211>    21
<212>    DNA
<213>    Artificial Sequence

<220>
<223>    utr 6


<400>    3
aaaactttgg atcaatcaac a                                                  21


<210>    4
<211>    21
<212>    DNA
<213>    Artificial Sequence

<220>
<223>    utr 7


<400>    4
ctctaatcac caggagtaaa a                                                  21


<210>    5

<211>    21
<212>    DNA
<213>    Artificial Sequence

<220>
<223>    utr 24


<400>    5
agaaaagctt tgagcagaaa c                                                21


<210>    6
<211>    21
<212>    DNA
<213>    Artificial Sequence

<220>
<223>    utr 35


<400>    6
aacactaaaa gtagaagaaa a                                                21


<210>    7
<211>    21
<212>    DNA
<213>    Artificial Sequence

<220>
<223>    U1 AAA


<400>    7
agagaagacg aaacacaaaa a                                                21


<210>    8
<211>    21
<212>    DNA
<213>    Artificial Sequence

<220>
<223>    U1 CCC


<400>    8
agagaagacg aaacacaacc c                                                21


<210>    9
<211>    21
<212>    DNA
<213>    Artificial Sequence

<220>
<223>    U1 GGG


<400>    9
agagaagacg aaacacaagg g                                                21


<210>    10
<211>    21
<212>    DNA
<213>    Artificial Sequence

<220>
<223>    U1 (-4,5G)


<400>    10
agagaagacg aaacacggaa g                                                21


<210>    11
<211>    21
<212>    DNA
<213>    Artificial Sequence

<220>
<223>    U1 (-4,5C)


<400>    11
agagaagacg aaacacccaa g                                                21


<210>    12
<211>    21
<212>    DNA
<213>    Artificial Sequence

<220>
<223>    UAAG


<400>    12
aagaagaaga agaagaagaa g                                                21


<210>    13
<211>    272
<212>    DNA
<213>    Artificial Sequence

<220>
<223>    BIP seq


<400>    13
atggctcgct cgtttggagc taacagtacc gttgtgttgg cgatcatctt cttcggtgag      60

tgattttccg atcttcttct ccgatttaga tctcctctac attgttgctt aatctcagaa     120

ccttttttcg ttgttcctgg atctgaatgt gtttgtttgc aatttcacga tcttaaaagg     180

ttagatctcg attggtattg acgattggaa tctttacgat ttcaggatgt ttatttgcgt     240

tgtcctctgc aatagaagag gctacgaagt ta                                   272


<210>    14
<211>    1524
<212>    DNA
<213>    Artificial Sequence

<220>
<223>    H5N1 HA gene


<400>    14
cacgccaaca actggaccga gcaggtggac accatcatgg agaagaacgt gaccgtgacc      60

cacgcccagg acatcctgga gaagacccac aacggcaagc tgtgcgacct ggacggcgtg 120

aagcccctga tcctgcgcga ctgcagcgtg gccggctggc tgctgggcaa ccccatgtgc 180

gacgagttca tcaacgtgcc cgagtggagc tacatcgtgg agaaggccaa ccccgccaac 240

gacctgtgct accccggcga cttcaacgac tacgaggagc tgaagcacct gctgagccgc 300

atcaaccact tcgagaagat ccagatcatc cccaagaaca gctggagcag ccacgaggcc 360

agcctgggcg tgagcagcgc ctgcccctac cagggcaaga gcagcttctt ccgcaacgtg 420

gtgtggctga tcaagaagaa caacgcctac cccaccatca agcgcagcta caacaacacc 480

aaccaggagg acctgctggt gctgtggggc atccaccacc ccaacgacgc cgccgagcag 540

acccgcctgt accagaaccc caccacctac atcagcgtgg gcaccagcac cctgaaccag 600

cgcctggtgc ccaagatcgc cacccgcagc aaggtgaacg gccagaacgg ccgcatggag 660

ttcttctgga ccatcctgaa gcccaacgac gccatcaact tcgagagcaa cggcaacttc 720

atcgcccccg agtacgccta caagatcgtg aagaagggcg acagcgccat catgaagagc 780

gagctggagt acggcaactg caacaccaag tgccagaccc ccatgggcgc catcaacagc 840

agcatgccct tccacaacat ccaccccctg accatcggcg agtgccccaa gtacgtgaag 900

agcaaccgcc tggtgctggc caccggcctg cgcaacagcc cccagcgcga gcgccgccgc 960

aagaagcgcg gcctgttcgg cgccatcgcc ggcttcatcg agggcggctg gcagggcatg 1020

gtggacggct ggtacggcta ccaccacagc aacgagcagg gcagcggcta cgccgccgac 1080

aaggagagca cccagaaggc catcgacggc gtgaccaaca aggtgaacag catcatcgac 1140

aagatgaaca cccagttcga ggccgtgggc cgcgagttca acaacctgga gcgccgcatc 1200

gagaacctga acaagaagat ggaggacggc ttcctggacg tgtggaccta caacgccgag 1260

ctgctggtgc tgatggagaa cgagcgcacc ctggacttcc acgacagcaa cgtgaagaac 1320

ctgtacgaca aggtgcgcct gcagctgcgc gacaacgcca aggagctggg caacggctgc 1380

ttcgagttct accacaagtg cgacaacgag tgcatggaga gcgtgcgcaa cggcacctac 1440

gactaccccc agtacagcga ggaggcccgc ctgaagcgcg aggagatcag cggcgtgaag 1500

ctggagagca tcggcaccta ccag 1524

<210> 15
<211> 502
<212> DNA
<213> Artificial Sequence

<220>
<223> CBD DNA seq

<400> 15
ttacacatgg catggatgaa ctatacaaat taggaggtgg aggtggaccc cgggcacacc 60

accaccacca ccactttcga agttcaccag tgcctgcacc tggtgataac acaagagacg 120

catattctat cattcaggcc gaggattatg acagcagtta tggtcccaac cttcaaatct 180

ttagcttacc aggtggtggc agcgccattg gctatattga aaatggttat tccactacct 240

ataaaaatat tgattttggt gacggcgcaa cgtccgtaac agcaagagta gctacccaga 300

atgctactac cattcaggta agattgggaa gtccatcggg tacattactt ggaacaattt 360

acgtggggtc cacaggaagc tttgatactt atagggatgt atccgctacc attagtaata 420

ctgcgggtgt aaaagatatt gttcttgtat tctcaggtcc tgttaatgtt gactggtttg 480

tattctcaaa tcaagaactt ag 502

## Description

### TECHNICAL FIELD

The present invention relates to a method for producing a transgenic plant which can produce a surface protein of avian influenza virus, hemagglutinin, in a highly efficient manner, a method of producing hemagglutinin protein which can induce an immune response against avian influenza virus,antigenic avian influenza virus from the transgenic plant, and a vaccine composition against avian influenza virus comprising hemagglutinin protein produced by the method.

### BACKGROUND ART

Avian influenza virus (AIV) is an RNA virus belonging to orthomyxovirus, whose spread is very fast. It has been known that the pathogenicity is very diverse from no clinical symptoms to almost 100% mortality following infection. All the avian influenza viruses belong to type A and have a variety of serotypes: hemagglutinin (HA) is classified into 16 subtypes, and neuraminidase (NA) into 9 subtypes. Depending on hemagglutinin and neuraminidase genes present on the surface of the virus, there are potentially 144 various combinations of the influenza virus type A.

Among these various subtypes of the influenza virus type A, the subtypes H5 and H7 are known to be pathogenic to birds, and the subtypes H1, H2, and H3 are known to cause influenza in humans. It has been generally known that avian influenza virus does not infect any animals, other than avian and swine species. However, patients infected with the avian influenza virus emerged in Hong Kong in 1997 and it was known that an H5N1 avian influenza virus caused the emergence of patients, which confirmed the possibility of human infection by the avian influenza virus. The human infection is thought to be caused by highly pathogenic virus generated by genetic combination between avian influenza virus and human influenza virus when they simultaneously infect human. Also in South Korea, a total of 19 outbreaks of H5N1, highly pathogenic avian influenza, occurred between December 2003 and March 21^{th}, 2004, affecting not only domestic poultry farming industry but also the related secondary industry owing to shrinking consumer confidence caused by concern about human infection. These outbreaks also caused enormous economic damage including, at the direct expense of the government, 150 billion won for the eradication of highly pathogenic avian influenza, and then came to an end. Recently, human cases of H5N1 infection have also persistently occurred in Thailand and Vietnam and evoked concern the world over.

However, avian influenza viruses have many various serotypes and there is weak or no cross-immunity among serotypes mutually. Thus, it is hard to prevent infection by other serotypes. Since avian influenza viruses are highly apt to undergo mutation, there is no effective vaccine for preventing avian influenza. Currently, the most effective prevention method is washing with antiseptic agents, and parenteral vaccination with inactivated influenza virus vaccine or a recombinant fowl pox virus vaccine. However, such methods are used only after the outbreak of avian influenza and examination of the virus subtype. Therefore, there are limitations for reducing or preventing the spread of avian influenza.

While worldwide huge amounts of money has been invested in avian influenza and development of various therapeutic agents and vaccines has been accelerated, enough vaccine production amounts to manage H5N1 virus effectively have not yet been attained. As of June, 2008, three H5N1 vaccines have been developed, but are still in the experimental stage, and the production amounts are very small. Thus, when a sharp rise in demand is needed, such as at a pandemic, the possibility to supply sufficient quantities of these vaccines is very low.

Particularly, there are limitations that the production of vaccines against H5N1 is difficult due to various reasons and it takes much time to commercialize them. Most vaccines against H5N1 are virus vaccines, which are obtained by the representative method in which viruses are inoculated generally in eggs, then amplified, and then isolated. However, the method has limitations that the production amount is small; the ability to manage a sharp rise in demand, such as a pandemic, is limited; and much money and time are required to install production facilities. Thus, it is realistically impossible to use eggs to produce highly pathogenic H5N1 vaccines. It is expected that vaccines not made from viruses but made from protein antigens may effectively prevent economic losses of poultry farmers and protect poultry and livestock farmers from fatal viruses. Therefore, there is a need to develop vaccines using protein antigens.

Meanwhile, technologies to use plants and produce useful physiological active substances from plants have recently been developed. Production of useful physiological active substances from plants have the following advantages: the unit cost of production can be substantially reduced; contamination sources, including viruses, oncogenes, enterotoxins, which may be generated during separation and purification of proteins synthesized from animal cells or microorganisms may be forestalled; when the biologically active substances are commercialized, long-term storage is possible for seeds, and if the plant are easily cultivated, the worldwide dissemination is possible in a seed state, and thus, it is more advantageous than proteins which should depend on refrigerated and frozen distribution; and if there is a sharp rise in demand for the biologically active substances, technologies or costs required for the mass production facilities are absolutely smaller than those of animal cell systems and it may be possible to provide a supply depending upon much demand in the shortest period.

Because plants have a eukaryotic protein synthesis pathway (post-translational modifications essential for mammals) plants are able to produce proteins similar to those expressed in mammals. For that reason, much of the focus has been placed on the production of useful physiological active substances from transgenic plants.

However, so far, technologies to synthesize and produce useful biologically active substances, for example, medically useful proteins, vaccines, and industrially valuable enzymes from plants efficiently have not become highly successful.

### DISCLOSURE OF THE INVENTION

### TECHNICAL PROBLEM

Thus, the present inventors have studied to develop a protein vaccine against avian influenza, especially against highly pathogenic virus H5N1 using the said characteristics of plants and found that the mass production of an antigenic protein of H5N1 virus, hemagglutinin (HA) from transgenic plants can be achieved by transforming plants using a recombinant vector for plant transformation comprising hemagglutinin (HA) gene of H5N1 virus. Then, the present inventors have developed an avian influenza vaccine derived from plants which is more economical and safer than conventional vaccines and can be used as an edible vaccine which is easy to administer, thereby leading to completion of the present invention.

Therefore, one object of the present invention is to provide a method of preparing a transgenic plant producing a hemagglutinin (HA), which is an antigenic protein of H5N1 virus.

Another object of the present invention is to provide a transgenic plant prepared by the method, wherein the transgenic plant produces an antigenic hemagglutinin (HA) protein of H5N1 virus.

Still another object of the present invention is to provide a method of producing an antigenic hemagglutinin (HA) protein of H5N1 virus, from the transgenic plant according to the present invention.

Even another object of the present invention is to provide a vaccine protein against H5N1 virus produced by the present invention.

Yet another object of the present invention is to provide a vaccine composition against H5N1 virus comprising the transgenic plant according to the present invention, hemagglutinin (HA) protein of H5N1 virus produced from the transgenic plant, or protein extracts of the transgenic plant.

Further another object of the present invention is to provide a method of diagnosing whether an antibody in a clinical specimen is formed by the infection of H5N1 virus or administration of the vaccine according to the present invention, wherein the method comprises the following steps of: (a) collecting blood from a clinical specimen; (b) separating serum from the collected blood; and (c) adding an antibody against hemagglutinin (HA) and an antibody against cellulose-binding domain (CBD) to the separated serum to react.

### TECHNICAL SOLUTION

In order to achieve the objects, the present invention provides a method of preparing a transgenic plant producing an antigenic hemagglutinin (HA) protein of H5N1 virus, the method comprising introducing a vector for plant transformation into a plant, wherein the vector comprises a gene construct consisting of (i) a DNA fragment consisting of any one nucleotide sequence selected from the group consisting of SEQ ID NOs:1 to 12; (ii) BiP (chaperone binding protein) gene having nucleotide sequence of SEQ ID NO:13; (iii) a polynucleotide encoding hemagglutinin (HA) protein of H5N1 virus having nucleotide sequence of SEQ ID NO: 14, (iv) a polynucleotide encoding a cellulose-binding domain (CBD) having nucleotide sequence of SEQ ID NO: 15; and (v) a polynucleotide encoding HDEL (His-Asp-Glu-Leu) peptide, wherein the gene construct is operably linked to a promoter.

In one embodiment of the present invention, a method of introducing the vector for plant transformation into a plant may be any one selected from the group consisting of *Agrobacterium* sp.-mediated transformation, particle gun bombardment, silicon carbide whiskers, sonication, electroporation, and PEG (polyethylene glycol) precipitation.

In one embodiment of the present invention, the plant may be dicotyledonous plants selected from the group consisting of *Arabidopsis thaliana,* soybeans, tobaccos, eggplants, red peppers, potatoes, tomatoes, Chinese cabbages, Chinese radishes, cabbages, lettuces, peaches, pears, strawberries, watermelons, muskmelons, cucumbers, carrots and salaries; or monocotyledonous plants selected from the group consisting of rice, barley, wheat, rye, corn, sugarcane, oats and onions.

The present invention also provides the transgenic plant prepared by the method of the present invention, wherein the transgenic plant produces an antigenic hemagglutinin (HA) of H5N1 virus.

Furthermore, the present invention provides a method of producing an antigenic hemagglutinin (HA) protein of H5N1 virus from the transgenic plant, wherein the method comprises cultivating the transgenic plant according to the present invention and isolating and purifying the HA protein expressed from the transgenic plant into which a polynucleotide encoding hemagglutinin (HA) protein of H5N1, having nucleotide sequence of SEQ ID NO: 14. In one embodiment of the present invention, hemagglutinin (HA) protein, which is an antigenic protein of H5N1 virus and isolated and purified from the transgenic plant, may be in a form fused with cellulose-binding domain (CBD).

The present invention also provides a vaccine protein against H5N1 virus produced by the method of the present invention.

In one embodiment of the present invention, the vaccine protein may be a cellulose-binding domain (CBD)-fused hemagglutinin (HA) protein of H5N1 virus.

Furthermore, the present invention provides a vaccine composition against H5N1 virus comprising the transgenic plant according to the present invention, a hemagglutinin (HA) protein of H5N1 virus produced from the transgenic plant, or protein extracts of the transgenic plant.

The present invention also provides a method of diagnosing whether an antibody in a clinical specimen is formed by the infection of H5N1 virus or administration of the vaccine according to the present invention, wherein the method comprising the steps of:

(a) collecting blood from the clinical specimen;

(b) separating serum from the collected blood; and

(c) adding an antibody against hemagglutinin (HA) and an antibody against cellulose-binding domain (CBD) to the separated serum to react.

In one embodiment of the present invention, it can be determined that the antibody in the clinical specimen is formed by the administration of the vaccine composition if both the antibody against hemagglutinin (HA) and the antibody against cellulose-binding domain (CBD) are detected in the reaction of the step (c), and that the antibody in the clinical specimen is formed by the infection of H5N1 virus if only the antibody against hemagglutinin (HA) is detected.

### ADVANTAGEOUS EFFECTS

According to the present invention, the method of producing hemagglutinin protein of antigenic H5N1 avian influenza virus from a plant transformed using a plant transformation recombinant vector, wherein the vector can express the hemagglutinin protein of antigenic H5N1 avian influenza virus in a highly efficient manner and can transport proteins expressed in plants to the endoplasmic reticulum and enable the retention of the proteins in the endoplasmic reticulum so as to accomplish glycosylation which is necessarily required for antigen activity, enables the mass production of antigenic hemagglutinin proteins at a low cost. The recombinant vector used in the present invention has a cellulose-binding domain inserted therein so as to isolate proteins expressed in plants in a quick and easy manner. In addition, the transgenic plant according to the present invention can be taken in the form of feed additives etc., and therefore can be used as edible vaccines. Further, the antigenic hemagglutinin produced by the method induces immune response when administered to an animal model, and therefore can be used not only as protein vaccines for H5N1 avian influenza virus, but also as diagnostic reagents for avian influenza virus infection.

### BRIEF DESCRIPTION OF THE DRAWINGS

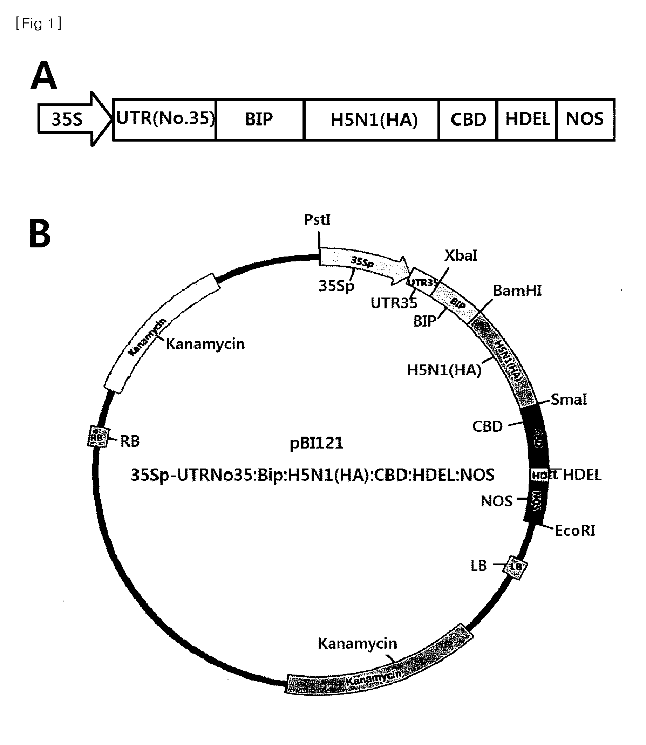

FIG. 1a is a schematic diagram illustrating the connecting form of the 35Sp-UTR35:Bip:H5N1(HA):CBD:HDEL:NOS part in the vector for plant transformation prepared in the present invention. FIG. 1b shows the cleavage map of the vector for plant transformation prepared in one embodiment of the present invention.

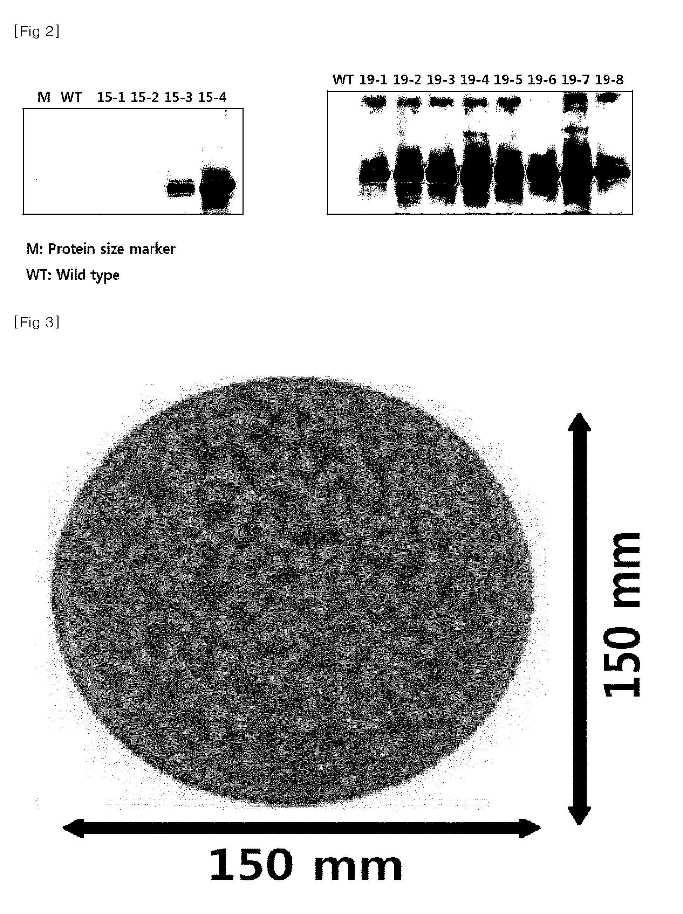

FIG. 2 shows the degree of HA expression of H5N1 from the transgenic plant through Western blot analysis using the CBD antibody after SDS-PAGE electrophoresis of proteins extracted from each transgenic plant produced by using the vector for plant transformation according to the present invention.

FIG. 3 is a photograph showing *Arabidopsis thaliana* capable of producing HA protein of H5N1, produced by using the vector for plant transformation according to the present invention.

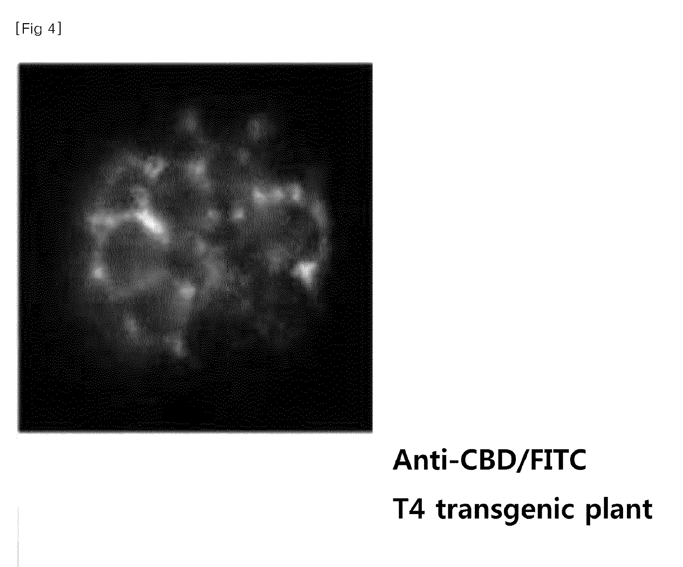

FIG. 4 is a photograph of the cellular position of HA antigenic protein of H5N1 expressed in the transgenic *Arabidopsis thaliana* according to the present invention, observed by cellular immunostaining.

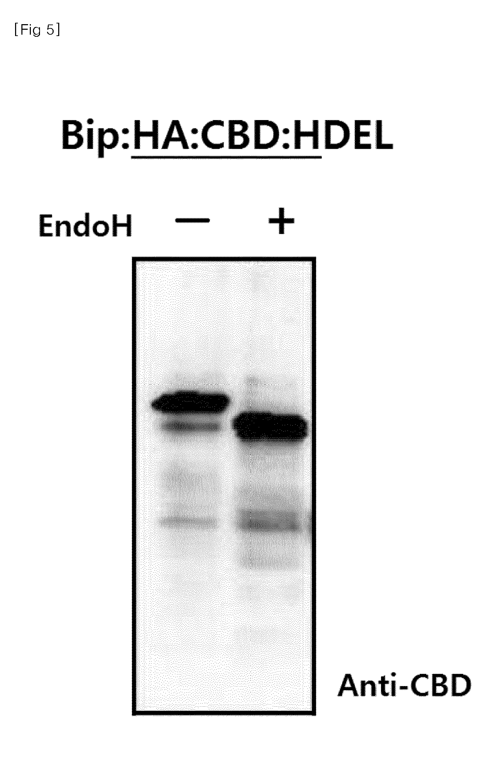

FIG. 5 is a result of Western blot analysis on the group in which protein extracts are treated with endoglycosidase H, the enzyme that degrades oligosaccharides, and the control group in which protein extracts are not treated with endoglycosidase H, to confirm whether HA antigenic proteins of H5N1 are glycosylated or not, after extracting proteins from the transgenic *Arabidopsis thaliana* according to the present invention.

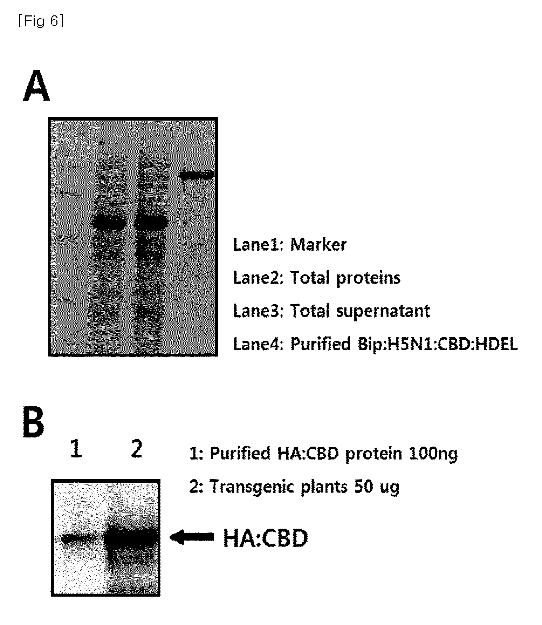

FIG. 6a shows a result of the confirmation of isolated and purified proteins by Coomassie staining. Antigenic HA proteins of H5N1 were isolated and purified using cellulose from the transgenic *Arabidopsis thaliana* according to the present invention. Then, SDS-PAGE electrophoresis and Coomassie staining were carried out.

FIG. 6b shows a result of measuring the amount of the expressed antigenic HA proteins of H5N1 among total water-soluble proteins extracted from the transgenic *Arabidopsis thaliana* according to the present invention. Western blot analysis using the CBD antibody was carried out and the signal intensity was measured using a multigauge program.

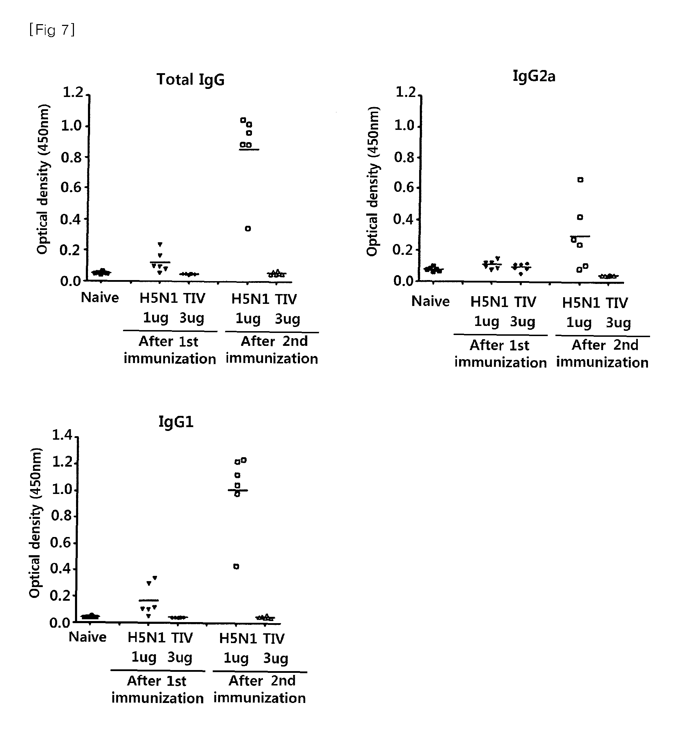

FIG. 7 shows results of examining the degree of formation of specific antibodies against avian influenza antigen, i.e. IgG, IgG2a, and IgG1 antibodies using each serum by ELISA. Antigenic HA proteins of H5N1 isolated from the transgenic *Arabidopsis thaliana* according to the present invention was injected into mice. For the control group, serum samples of mice injected with TIV instead of antigenic HA proteins of H5N1 were used.

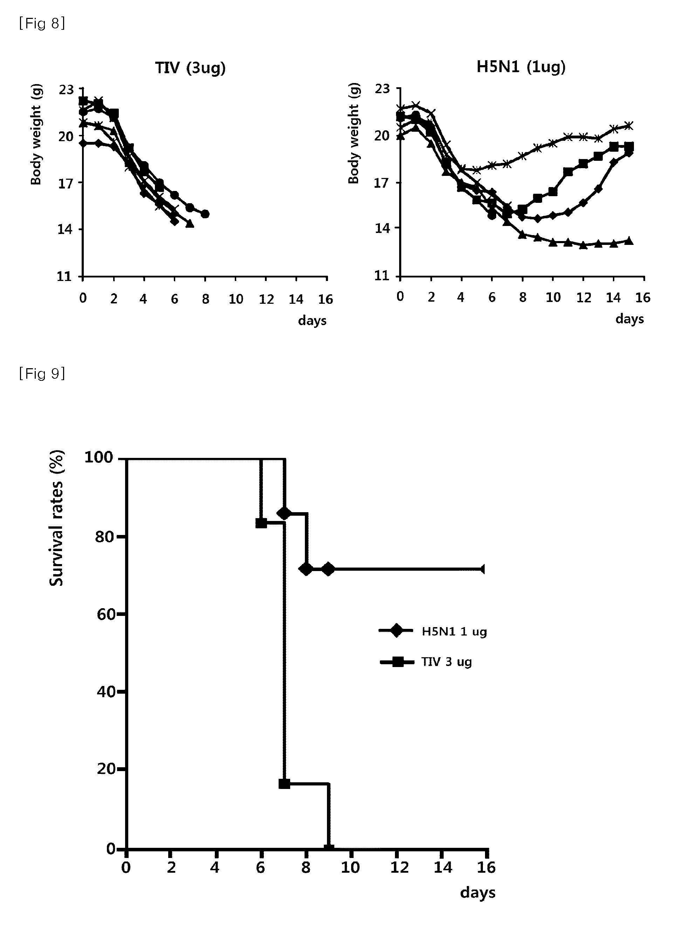

FIG. 8 is a graph which measured change in the body weight of each mouse, after the injection of antigenic HA protein of H5N1 produced according to the present invention or TIV for the control group into mice and induction of the infection with H5N2 virus.

FIG. 9 is a graph showing the survival rate of each mouse, after the injection of antigenic HA protein of H5N1 produced according to the present invention or TIV for the control group into mice and induction of the infection with H5N2 virus.

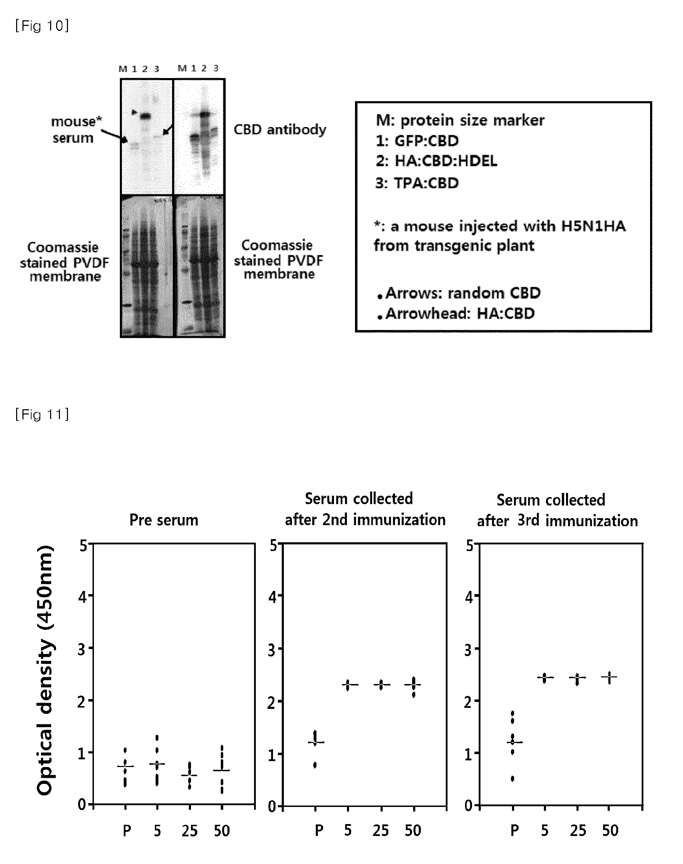

FIG. 10 shows a result of the confirmation of the formation of the antibody against HA of H5N1 as well as the antibody against CBD using the serum of the mouse through Western blot analysis. Mice were immunized with antigenic HA protein of H5N1 produced according to the present invention.

FIG. 11 shows results of examining the formation of the specific antibody against avian influenza antigen, anti-total IgG (chicken) antibody by ELISA method. Antigenic HA protein of H5N1 isolated from the transgenic *Arabidopsis thaliana* according to the present invention was injected into chickens. Serum samples were separated and the formation of the specific antibody against avian influenza antigen was examined by ELISA method. For the control group, results were shown in which serum samples of chickens injected with PBS buffer instead of antigenic HA protein of H5N1 were used to perform the antigen-antibody reaction.

### BEST MODE FOR CARRYING OUT THE INVENTION

The present invention relates to the development of a vaccine for the prevention of H5N1 virus, the highly pathogenic avian influenza, and is characterized in the preparation of a transgenic plant producing hemagglutinin (HA), an antigenic protein of H5N1 virus. More specifically, the present invention provides a method of preparing a transgenic plant producing an antigenic hemagglutinin (HA) protein of H5N1 virus, wherein the method comprises introducing a vector for plant transformation into a plant, wherein the vector comprises a gene construct consisting of (i) a DNA fragment consisting of any one nucleotide sequence selected from the group consisting of SEQ ID NOs:1 to 12; (ii) BiP (chaperone binding protein) gene having nucleotide sequence of SEQ ID NO: 13; (iii) a polynucleotide encoding hemagglutinin (HA) protein of H5N1 virus, having nucleotide sequence of SEQ ID NO: 14, (iv) a polynucleotide encoding a cellulose-binding domain (CBD), having nucleotide sequence of SEQ ID NO: 15; and (v) a polynucleotide encoding HDEL (His-Asp-Glu-Leu) peptide, wherein the gene construct is operably linked to a promoter.

In order to maximize the production of a useful protein, i.e. hemagglutinin (HA) protein of H5N1 virus, which can be used for a vaccine from plants, the present inventors prepared a recombinant vector for plant transformation which introduces a gene encoding the HA protein into a plant and express in it. The recombinant vector comprises a nucleotide sequence of a 5' untranslated region (5' UTR) which can regulate protein expression at the translation stage. It has been known that generally 5' untranslated regions (5' UTR) of mRNA play an important role in post-transcriptional regulation of gene expression, regulation of mRNA transportation out of the nucleus, the regulation of the efficiency of protein translation, and the regulation of mRNA stability.

In the present invention, the 5' untranslated region (5' UTR) may be a DNA fragment which improves the translational efficiency of proteins which are isolated from *Arabidopsis thaliana,* and preferably has any one nucleotide sequence selected from the group consisting of SEQ ID NOs: 1 to 12 represented by the following table.

5' UTR Sequences

| 5' UTR No. | nucleotide sequence | SEQ ID NO. |
|---|---|---|
| UTR 1 | AGAGAAGACGAAACACAAAAG | 1 |
| UTR 2 | GAGAGAAGAAAGAAGAAGACG | 2 |
| UTR 6 | AAAACTTTGGATCAATCAACA | 3 |
| UTR 7 | CTCTAATCACCAGGAGTAAAA | 4 |
| UTR 24 | AGAAAAGCTTTGAGCAGAAAC | 5 |
| UTR 35 | AACACTAAAAGTAGAAGAAAA | 6 |
| U1 AAA | AGAGAAGACGAAACACAAAAA | 7 |
| U1 CCC | AGAGAAGACGAAACACAACCC | 8 |
| U1 GGG | AGAGAAGACGAAACACAAGGG | 9 |
| U1(-4, 5G) | AGAGAAGACGAAACACGGAAG | 10 |
| U1(-4, 5C) | AGAGAAGACGAAACACCCAAG | 11 |
| U AAG | AAGAAGAAGAAGAAGAAGAAG | 12 |

In addition, the recombinant vector for plant transformation according to the present invention may comprise a targeting gene to move the hemagglutinin protein to endoplasmic reticulum, in order to mass-produce the hemagglutinin (HA) protein of antigenic H5N1 virus from plants.

Generally, when heterologous proteins are overexpressed in transgenic plants or cells, proteolytic degradation often occurs. However, if foreign proteins are targeted to various intracellular organelles, these proteins can be stored more stably. Particularly, when a heterologous protein is targeted to the endoplasmic reticulum rather than being in the cytoplasm, proteolytic degradation can be minimized.

In addition, most viral surface proteins exist in a glycosylated form and it is known that whether viral surface protein is glycosylated or not plays a very important role in the formation of stable structure of viral protein, induction of antibody reaction, and induction of immune response, etc. (Goffard, A., et al., J. Virol. 79, 8400-8409, 2005; Hebert, D. N., et al., J. Cell Biol. 139, 613-623, 1997). It has also been reported that glycosylation of viral proteins help antibody formation when glycosylated proteins and non-glycosylated proteins are compared (Ewasyshyn, M., et al., J. Gen. Virol. 74, 2781-2785, 1993). Since the endoplasmic reticulum contains large amounts of mannoses and glycosylation occurs actively in the endoplasmic reticulum, moving hemagglutinin (HA) proteins of H5N1 virus to the endoplasmic reticulum can maintain stability of the proteins and obtain large amounts of HA proteins in a glycosylated form.

In the present invention, a polynucleotide encoding Bip (chaperone binding protein) may be used to move hemagglutinin (HA) proteins of H5N1 virus to the endoplasmic reticulum within plants. Preferably, instead of cDNA of Bip, the polynucleotide having nucleotide sequence of SEQ ID NO: 13, a genomic DNA of Bip comprising intron, may be used.

The Bip, the luminal binding protein, was identified as the immunoglobulin heavy chain binding protein and the glucose regulated protein, and is a member of the HSP70 chaperone family localized to the endoplasmic reticulum and binds transiently to newly synthesized proteins in the endoplasmic reticulum. Signal sequences which determine targeting to the endoplasmic reticulum are contained at the N-terminal of Bip and play a role in targeting target proteins to the endoplasmic reticulum.

The recombinant vector for plant transformation may comprise a polynucleotide encoding an ER retention signal peptide such as HDEL to ensure that in case of the HDEL signal peptide, heterologous proteins are retained in the endoplasmic reticulum to increase folding and assembly by molecular chaperones and consequently further minimize proteolytic degradation (Nuttall, J. *et al.,* 2002). For example, it has been known that when heterologous proteins were retained in the endoplasmic reticulum rather than sent to the secretory pathway, the yield of heterologous proteins increased by about 10 to 100 times (Hellwig, S. *et al.,* 2004).

In addition, the recombinant vector for plant transformation according to the present invention may comprise a gene encoding hemagglutinin (HA) present on the surface of H5N1 virus. Generally, influenza viruses have hemagglutinin (HA) and neuraminidase (NA) antigens on their surface. HA and NA are relatively large glycoproteins. HA is a protein which helps the viruses recognize and penetrate cell surfaces. NA is a protein which helps the viruses escaping from host cells after the viruses proliferate in the host cells. It has been known that HA and NA are proteins which cause immune responses in host cells. Particularly, HA protein has the ability to agglutinate red blood cells of birds and is known to be an antigen which causes most antibody responses triggered in the body.

Therefore, in the present invention, in order to produce a vaccine against H5N1 virus from plants, a polynucleotide encoding hemagglutinin (HA) protein of H5N1 virus, preferably hemagglutinin (HA) gene of H5N1 virus having nucleotide sequence of SEQ ID NO: 14 may be comprised in the recombinant vector for plant transformation. The polynucleotide having nucleotide sequence of SEQ ID NO: 14 encodes a polypeptide comprising a part of the hemagglutinin protein of H5N1 type/Hong Kong/213/03 viral strain, wherein the part excludes 23 amino acids of the HA protein.

Furthermore, the recombinant vector for plant transformation may comprise a polynucleotide encoding a cellulse-binding domain (CBD). The polynucleotide encoding CBD comprised in the recombinant vector may be any nucleotide sequence which is generally used for protein purification in the art, and preferably may have nucleotide sequence of SEQ ID NO: 15.

Therefore, the recombinant vector for plant transformation according to the present invention is an expression vector comprising a gene construct consisting of a DNA fragment which can improve the translational efficiency of heterologous protein, Bip gene sequence, the polynucleotide encoding HA protein of H5N1 virus, a polynucleotide encoding CBD, and a polynucleotide encoding HDEL peptide serially and the said gene construct may be operably linked to a promoter.

In the present invention, the term "expression vector" refers to a plasmid, a virus, or other vehicle known in the art that may be manipulated by insertion or incorporation of the DNA fragments, the gene constructions, or the polynucleotide according to the present invention. The DNA fragments, the gene constructs, or the polynucleotide according to the present invention may be operably linked to an expression control element. The operably linked gene construct and the expression control element may be comprised in one expression vector which comprises a selectable marker and a replication origin together. The term "operably linked" may refer to a gene and an expression control element linked in a manner to allow the gene expression when a suitable molecule binds to the expression control element. The term "expression control element" refers to a DNA fragment which controls the expression of a polynucleotide operably linked in a specific host cell. The control element may comprise a promoter for performing transcription, an arbitrary operator element for regulating transcription, a suitable mRNA ribosome binding site, and DNA fragments for controlling transcription and translation termination.

Any promoter may be used provided that it can express inserted gene in plants, and is not particularly restricted. Examples of promoters include, but are not limited to, 35S RNA and 19S RNA promoters of CaMV, a full length transcription promoter derived from figwort mosaic virus (FMV), and a promoter of TMV coat protein. Examples of the suitable vector for incorporation of the DNA fragments, the gene constructs, or the polynucleotides according to the present invention, into plant cells, include Ti plasmids, plant virus vectors, *etc.* Preferred examples of the suitable vectors include, but are not limited to, binary vectors such as pCHF3, pPZP, pGA, and pCAMBIA vectors. Any vector may be used in the present invention, provided that it can introduce the said polynculeotides according to the present invention into plant cells.

In one embodiment of the present invention, the recombinant vector for the expression of antigenic hemagglutinin of H5N1 virus was prepared using a vector developed by the present inventors, which can move a heterologous gene to the endoplasmic reticulum in plants to express and maintain, i.e., the vector disclosed in Korean Patent Application No. 2009-0081403, as a base vector, by substituting the heterologous gene of the base vector with a polynucleotide encoding hemagglutinin protein of H5N1 virus having nucleotide sequence of SEQ ID NO: 14; cutting a region which contains 35S promoter and the NOS terminator part; and linking the region to pBI121 vector which is a vector for plant transformation using PstI and *Eco*RI restriction enzymes. A plasmid map of the gene construct consisting of the promoter, UTR sequence, Bip sequence, H5N1 HA sequence, CBD sequence, and HDEL sequence in the vector prepared according to the present invention was shown in FIG. 1. The Korean Patent Application No. 2009-0081403 is herein entirely incorporated by reference. Therefore, the present invention can provide the method of preparing a transgenic plant producing hemagglutinin, an antigenic protein of H5N1 virus comprising the step of introducing the recombinant vector for plant transformation described before into a plant.

The method of introducing the recombinant vector of the present invention into a plant may be, but is not limited to, Agrobacterium sp.-mediated transformation, particle gun bombardment, silicon carbide whiskers, sonication, electroporation, and PEG (polyethylene glycol) precipitation. In one embodiment of the present invention, *Arabidopsis thaliana* was transformed with the recombinant vector of the present invention by Agrobacterium sp.-mediated transformation and a photograph of *Arabidopsis thaliana* transformed by the method was shown in FIG. 3.

Therefore, the present invention provides the transgenic plant prepared by the method according to the present invention, wherein the transgenic plant produces an antigenic hemagglutinin protein of H5N1 virus.

In addition, the plant transformed with the recombinant vector of the present invention, i.e., the plant which can produce hemagglutinin of H5N1 in a highly efficient manner, because the antigenic hemagglutinin (HA) protein of H5N1 virus, is translated in a highly efficient manner and accumulated in the endoplasmic reticulum in the plant, may be obtained by either sexual or asexual propagation, that is a conventional method in the art. More specifically, the plant of the present invention may be obtained by sexual propagation, a process by which plants reproduce from seeds which are produced through the pollination of flowers. In addition, the plant of the present invention may be obtained by transforming plants using the recombinant vector according to the present invention and then performing asexual propagation which is a process comprising callus induction, rooting, and acclimation to soil according to conventional methods. That is, explants of the plant transformed with the recombinant vector according to the present invention are transferred to a suitable culture medium that is known in the art, and cultured to induce the formation of callus. When shoots are formed, they are transferred and cultured in a culture medium without hormones. After about two weeks, the shoots are transferred to a rooting medium to induce roots. After roots are induced, they are transferred to soil and acclimated, and then the plant according to the present invention can be obtained. In the present invention, the transgenic plant may include not only a whole plant but also tissues, cells, and seeds, which are obtained from the whole plant.

In the present invention, the plant may be dicotyledonous plants or monocotyledonous plants. Examples of dicotyledonous plants may be, but are not limited to, *Arabidopsis thaliana,* soybeans, tobaccos, eggplants, red peppers, potatoes, tomatoes, Chinese cabbages, Chinese radishes, cabbages, lettuces, peaches, pears, strawberries, watermelons, muskmelons, cucumbers, carrots and salaries. Examples of monocotyledonous plants may be, but are not limited to, rice, barley, wheat, rye, corn, sugarcane, oats and onions.

Meanwhile, in order to identify whether hemagglutinin protein of H5N1 is located in the endoplasmic reticulum in the cell of the plant transformed with the recombinant vector, the present inventors examined in one embodiment using cellular immunostaining and found that hemagglutinin protein of H5N1 existed in the endoplasmic reticulum in the plant (FIG. 4).

According to one embodiment of the present invention, in order to identify whether hemagglutinin protein of H5N1 expressed in the transgenic plant exists in a glycosylated form or not, the present inventors extracted proteins from the plant and treated endoglycosidase H, the enzyme that degrades oligosaccharides. As a result, it was found that the expressed hemagglutinin protein of H5N1 was glycosylated through post-translational modification (FIG. 5).

Furthermore, the present invention may provide a method of producing an antigenic hemagglutinin (HA) protein of H5N1 virus, from a plant transformed with the recombinant vector according to the present invention. The method may comprise cultivating the transgenic plant and isolating and purifying the protein which is expressed from the plant into which a polynucleotide encoding antigenic hemagglutinin (HA) protein of H5N1 virus is introduced, wherein the polynucleotide has nucleotide sequence of SEQ ID NO: 14.

More specifically, the method of producing an antigenic hemagglutinin (HA) protein of H5N1 virus, from the transgenic plant may be accomplished by introducing the recombinant vector according to the present invention into a plant or by transforming plant cells with the recombinant expression vector, then cultivating the plant or plant cells for suitable time periods to express hemagglutinin (HA), and then obtaining hemagglutinin (HA) from the transgenic plant or plant cells. Any method of expressing the protein may be used provided that it has been known in the art. Collection of highly-expressed proteins in the transgenic plant or plant cells may be carried out through various isolation and purification methods that are known in the art. Generally, centrifugation of the cell lysate, followed by precipitation, for example, salting out precipitation (ammonium sulfate precipitation and sodium phosphate precipitation), solvent precipitation (precipitation of protein fraction using acetone, ethanol, *etc.*) may be carried out to remove cell debris *etc.* Dialysis, electrophoresis, various column chromatographies, *etc.* may be carried out. Ion-exchange chromatography, gel-filtration chromatography, HPLC, reverse phase-HPLC, affinity column chromatography, ultrafiltration, etc. may be applied, alone or in combination, to purify hemagglutinin (HA) protein of the present invention (Maniatis et al., Molecular Cloning: A Laboratory Manual, Cold Spring Harbor Laboratory, Cold Spring Harbor, N.Y.(1982); Sambrook et al., Molecular Cloning: A Laboratory Manual, 2d Ed., Cold Spring Harbor Laboratory Press(1989); Deutscher, M., Guide to Protein Purification Methods Enzymology, vol. 182. Academic Press. Inc., San Diego, CA (1990)).

In addition, in the present invention, in order to isolate and purify hemagglutinin (HA) protein easily and quickly from the transgenic plant, the polynucleotide which encodes a cellulose-binding domain (CBD) of cellulase, preferably having nucleotide sequence of SEQ ID NO: 15 may be additionally operably linked to the recombinant vector of the present invention described before. Accordingly, the antigenic hemagglutinin (HA) protein of H5N1 virus expressed in the plant, may be in a fused form with cellulose-binding domain (CBD). Therefore, it can be easily isolated and purified using a chromatography which uses a cellulose carrier as a column.

Therefore, the present invention can provide a recombinant hemagglutinin protein of H5N1 virus having antigenicity, which is produced by the above method. The recombinant HA protein produced by the method may be a vaccine protein against H5N1 virus. The protein may be a fusion protein in which the hemagglutinin protein of H5N1 virus is fused to cellulose-binding domain (CBD).

Furthermore, the present inventors investigated whether the H5N1 hemagglutinin fusion protein produced from the plant by the method of the present invention as described above really acts as an antigen and can be used for a H5N1 virus vaccine or not.

That is, according to one embodiment of the present invention, the antigenic hemagglutinin protein of H5N1 virus which was isolated and purified by the method of the present invention described above was injected to a mouse model intramuscularly, and then blood was collected. Serum was separated and antigen-specific antibody response was investigated using ELISA method. For the control group, instead of hemagglutinin antigenic protein, TIV (trivalent influenza vaccine; H1N1 + H3N2 + B type HA protein) was used. Consequently, TIV used for the control group was not able to induce H5N1-specific antibody response, but the experimental group in which mice were administered with the hemagglutinin antigenic protein of H5N1 produced in the present invention, had an immune response and induced the antigen-specific antibody response (FIG. 8).

From the result, the present inventors found that the hemagglutinin protein of H5N1 virus of the present invention, which was produced from the plant, can induce antibodies against HA protein in a living body and then investigated whether the hemagglutinin protein of H5N1 virus really has a defensive effect against avian influenza virus or not. According to one embodiment of the present invention, mice injected with the hemagglutinin protein of H5N1 virus of the present invention, which was produced from the plant, or TIV (the control group) were infected with H5N2 virus which is known to be relatively low risk relative to H5N1. Change in body weight and the survival rate were examined. In the group administered with the H5N1 hemagglutinin protein, body weight of the mouse was reduced, but time passed and after 15 days, more than 90% of the initial body weight was recovered. However, in the group administered with TIV, body weight of the mouse decreased consistently (FIG. 8). According to the result of the measurement of the survival rate, the group administered with TIV, all mice died between 6th day and 8th day. In the group administered with the H5N1 hemagglutinin protein, one mouse died on 7th day and another mouse died on 8th day, and the survival rate was 67% in comparison with the control group (FIG. 9).

Therefore, the present inventors found that the hemagglutinin protein of H5N1 virus produced from the transgenic plant according to the present invention can not only induce antibody formation against H5N1 virus, but also has a defensive effect against avian influenza virus.

As described above, from the experiments on mice, the present inventors found that the antigenic hemagglutinin protein of H5N1 virus produced from the plant by the method of the present invention is immunogenic and can induce the antigen-specific antibody response. Based on the result, the present inventors found that the hemagglutinin protein of H5N1 virus produced in the present invention can be used as a vaccine applicable to mammals, including mice and humans.

In order to investigate whether the hemagglutinin protein of H5N1 virus produced from the plant by the method of the present invention can be used as a vaccine applicable to not only mammals but also birds and poultry, the present inventors administered the antigenic hemagglutinin protein of H5N1 virus, produced from the plant by the method of the present invention to chickens in one embodiment of the present invention. Serum samples were separated and antigen-specific antibody response was investigated using ELISA method. For the control group, instead of the antigenic hemagglutinin protein, PBS buffer was administered. Consequently, chickens administered with PBS buffer could not induce H5N1-specific antibody response. However, chickens administered with the antigenic hemagglutinin protein of H5N1 produced in the present invention had an immune response and induced antigen-specific antibody response regardless of sex (FIG. 11).

Therefore, it was found that the hemagglutinin protein of H5N1 virus produced from the transgenic plant according to the present invention can be used for the manufacture of a vaccine applicable to not only mammals but also birds and poultry, including chickens.

Accordingly, the present invention can provide the transgenic plant according to the method of the present invention described above, the antigenic hemagglutinin protein of H5N1 virus produced from the transgenic plant, or a vaccine composition against H5N1 virus comprising protein extracts of the transgenic plant.

The vaccine composition according to the present invention may be administered to an individual who needs it through various ways including oral administration, percutaneous administration, subcutaneous administration, intravenous administration, or intramuscular administration. The administration dose of the composition may be controlled appropriately depending on various factors such as administration pathway, body weight, and age of the individual, etc. The composition according to the present invention may be administered in combination with other known compounds which have the preventive or therapeutic effect on the infection of H5N1 virus and may further comprise pharmaceutically acceptable carriers, diluents, or excipients. The vaccine composition of the present invention may comprise from about 100 to about 1,000 mg/L of the hemagglutinin protein of H5N1 virus of as an active ingredient and the administration dose may be from about 50 to 500 mg, but not limited to such.

Examples of the carriers, excipients, and diluents which may be comprised additionally in the vaccine composition include lactose, dextrose, sucrose, sorbitol, mannitol, xylitol, erythritol, maltitol, starch, acacia gum, alginate, gelatin, calcium phosphate, calcium silicate, cellulose, methylcellulose, polyvinylpyrrolidone, water, methyl hydroxybenzoate, propyl hydroxybenzoate, talc, magnesium stearate, and mineral oil. Moreover, fillers, anticoagulants, lubricants, wetting agents, aromatics, emulsifiers, preservatives, etc. may be comprised additionally.

The composition of the present invention may be formulated by known methods in the art in order to provide a rapid, continuous, or delayed release of active ingredients after the administration to the individual who needs to it. The formulation may be, but is not limited to, powders, granules, tablets, emulsions, syrups, aerosols, soft or hard gelatin capsules, sterile injection solution, and sterilized powders.

The individual to which the vaccine composition of the present invention may be administered may include all individuals who may be objects of H5N1 virus infection, such as mammals including humans, birds, livestock, poultry, etc.

Furthermore, the transgenic plant of the present invention may be administered in itself or by grinding the plant and adding it to animal feeds or drinking water, and thus, the transgenic plant may be used for the vaccine for oral administration.

Accordingly, the present invention can provide the transgenic plant according to the present invention, the hemagglutinin (HA) protein of H5N1 virus produced from the transgenic plant, or the vaccine composition for oral administration against H5N1 virus comprising protein extracts from the transgenic plant. When the transgenic plant are used as the vaccine composition for oral administration, there are following advantages: the possibility of contamination by animal viruses is low; since the produced hemagglutinin (HA) protein of H5N1 virus is contained in the plant, long-term storage and transportation are practicable; and the cost of injection inoculation can be significantly reduced.

Since it is possible to isolate and purify the hemagglutinin (HA) antigenic protein of H5N1 virus, highly pathogenic avian influenza, from the transgenic plant according to the present invention, the present invention can provide a diagnostic reagent for H5N1 virus infection. Furthermore, the present invention can provide a method of diagnosis of H5N1 virus infection by allowing the isolated and purified antigenic hemagglutinin (HA) protein of H5N1 virus to react with serum, *etc.* of poultry or a mammal.

Besides, the antigenic hemagglutinin (HA) protein of H5N1 virus, which is isolated and purified from the transgenic plant according to the present invention, has a characteristic of a fusion protein that is fused with cellulose-binding domain (CBD).

Thus, the present inventors examined whether an antibody against the hemagglutinin protein-fused CBD protein is formed or not, when the hemagglutinin (HA) antigenic protein of H5N1 virus produced by the present invention is administered to a mouse. Consequently, it was found that when the HA antigenic protein was administered, the antibody against the fused CBD protein was also formed (FIG. 10).

Therefore, based on the result, the present invention can diagnose whether the antibody against hemagglutinin (HA) formed in the individual is formed by H5N1 viral infection or by the vaccine comprising the hemagglutinin (HA) of H5N1 virus according to the present invention through the process of detecting the antibody against hemagglutinin (HA) of H5N1 virus and the antibody against CBD protein from serum obtained in the individual.

Therefore, the present invention can provide a method of diagnosing whether an antibody in a clinical specimen is formed by the infection of H5N1 virus or administration of the vaccine, the method comprising the steps of:

(a) collecting blood from the clinical specimen;

(b) separating serum from the collected blood; and

(c) adding an antibody against hemagglutinin (HA) and an antibody against cellulose-binding domain (CBD) to the separated serum to react.

More specifically, blood samples are collected from a clinical specimen, preferably an object who has the chance of the infection by avian influenza virus, for example, from all kinds of animals except for birds or humans; serums samples are separated from bloods using conventionally used methods in the art; and then the antibody against hemagglutinin (HA) and the antibody against cellulose-binding domain (CBD) are added to generate an immune reaction. Then, through the detection of the antibody against hemagglutinin (HA) and the antibody against cellulose-binding domain (CBD), the diagnosis method can diagnose if the hemagglutinin (HA) antibody formed in the clinical specimen is formed by the H5N1 virus infection or by the vaccine comprising the hemagglutinin (HA) of H5N1 virus according to the present invention.

In the diagnosis according to the immune reaction, if both the antibody against hemagglutinin and the antibody against cellulose-binding domain (CBD) are detected from the serum obtained from the clinical specimen, it can be determined that the HA antibody of H5N1 virus is formed by administration of the vaccine according to the present invention. If only the antibody against hemagglutinin is detected from the serum obtained from the clinical specimen, it can be determined that the HA antibody of H5N1 virus is formed by the H5N1 virus infection.

Thus, the diagnosis method according to the present invention has the following effects: after the administration of the vaccine or the vaccine composition of the present invention, it can be determined whether the antibody against H5N1 virus is formed effectively or not; if the vaccine against avian influenza is inoculated and it is diagnosed whether the HA antibody against H5N1 virus is formed by the H5N1 virus infection through the diagnosis method, preventive or treating measures can be taken quickly, and thus, unnecessary destruction of poultry can be prevented and economical losses can be reduced; and the human infection can be prevented or treated. Therefore, the antigenic H5N1 hemagglutinin protein in a form fused with CBD produced in the present invention can be used as a marker vaccine.

Hereinafter, the present invention will be described in more detail with reference to the following examples. However, the following examples are provided for illustrative purposes only, and the scope of the present invention should not be limited thereto.

**Example 1**

**Preparation of the vector for plant transformation comprising the antigenic gene of H5N1 virus**

In order to produce the vector for plant transformation comprising the antigenic hemagglutinin (HA) gene on the surface of the HPAI (H5N1) virus, the present inventors used the recombinant vector comprising a DNA fragment for improving the translational efficiency, which improves the translational efficiency of a heterologous protein, developed by the present inventors and described in the Korean Patent Application No. 2009-0081403. That is, for the recombinant vector for plant transformation used in the present invention, the present inventors linked the region of a sequence comprising: cauliflower mosaic virus 35S promoter; the DNA fragment which improves the translational efficiency of heterologous protein, which is a 5' UTR (5' untranslated region) and a polynucleotide having nucleotide sequence of SEQ ID NO: 6 among polynucleotides having nucleotide sequences of SEQ ID NOs: 1 to 12 shown in the below Table 1; a polynucleotide encoding Bip (chaperone binding protein), having nucleotide sequence of SEQ ID NO: 13, which is able to target the heterologous protein to the endoplasmic reticulum (ER) in a plant cell; a polynucleotide encoding a antigenic hemagglutinin protein of H5N1 virus, having nucleotide sequence of SEQ ID NO: 14 (which is a part hemagglutinin protein of HPAI (H5N1) type A/Hong Kong/213/03 viral strain, excluding the last 23 amino acids); a polynucleotide sequence encoding a cellulose-binding domain (CBD), having nucleotide sequence of SEQ ID NO: 15; HDEL (His-Asp-Glu-Leu) peptide, the signal peptide which retains the heterologous protein within the endoplasmic reticulum; and a termination codon comprising 35S terminator (NOS), to a conventionally used vector, the pBI121 vector, using PstI and *Eco*RI restriction enzymes and prepared the vector for plant transformation, pBI121-35Sp-UTR35:Bip:H5N1(HA):CBD:HDEL:NOS. The gene construct map of 35Sp-UTR35:Bip:H5N1(HA):CBD:HDEL:NOS in the vector for plant transformation prepared according to the present invention is shown in FIG. 1a, and the plasmid map of the prepared vector is shown in FIG. 1b.

[Table 1] 5' UTR Sequences

| 5' UTR No. | nucleotide sequence | SEQ ID NO. |
|---|---|---|
| UTR 1 | AGAGAAGACGAAACACAAAAG | 1 |
| UTR 2 | GAGAGAAGAAAGAAGAAGACG | 2 |
| UTR 6 | AAAACTTTGGATCAATCAACA | 3 |
| UTR 7 | CTCTAATCACCAGGAGTAAAA | 4 |
| UTR 24 | AGAAAAGCTTTGAGCAGAAAC | 5 |
| UTR 35 | AACACTAAAAGTAGAAGAAAA | 6 |
| U1 AAA | AGAGAAGACGAAACACAAAAA | 7 |
| U1 CCC | AGAGAAGACGAAACACAACCC | 8 |
| U1 GGG | AGAGAAGACGAAACACAAGGG | 9 |
| U1(-4, 5G) | AGAGAAGACGAAACACGGAAG | 10 |
| U1(-4, 5C) | AGAGAAGACGAAACACCCAAG | 11 |
| U AAG | AAGAAGAAGAAGAAGAAGAAG | 12 |

**Example 2**

**Preparation of the transgenic plant which expresses HA of H5N1 virus**

*Arabidopsis thaliana* which expresses HA of H5N1 virus was prepared with the recombinant vector for plant transformation prepared in Example 1 using *Agrobacterium* sp.- mediated transformation, the method known in the art. The recombinant vector for plant transformation prepared in Example 1 used pBI121 as a base vector. Since pBI121 vector has a kanamycin resistance as selectable marker in plants, *Arabidopsis thaliana* transformed with the recombinant vector prepared by the method of the present invention was selected using kanamycin resistance test. From *Arabidopsis thaliana* selected with the kanamycin resistance test, the first generation transformant was established by selecting lines which express HA proteins well through Western blot analysis. Then in the second generation, lines whose ratio of dead individuals:survived individuals measured using a selectable marker was clearly 1:3 were selected as single copy transformants. Then, these lines were set as the third generation transformants. In the third generation, the lines in which all the individuals were survived and confirmed to express proteins by using Western blot analysis were selected as the homozygous lines. By maintaining these lines, the transgenic *Arabidopsis thaliana* plant strain was established. The Western blot analysis for confirming the expression of HA protein was carried out using the CBD antibody by a conventional Western blot analysis. The result showing the expression of hemagglutinin (HA) antigenic protein of H5N1 virus was shown in FIG. 2 and *Arabidopsis thaliana* transformed according to the present invention is shown in FIG. 3.

**Example 3**

**Examination of the location of hemagglutinin (HA) antigenic protein of H5N1 within the endoplasmic reticulum in the transgenic plant**

As described above, the present inventors used BiP, the signal sequence which transports the HPAI (H5N1) type A/Hong Kong/213/03 hemagglutinin (HA) protein to the endoplasmic reticulum (ER). Therefore, to examine whether the HA protein, which is expressed in the transgenic plant prepared in Example 2, is located in the endoplasmic reticulum, immunostaining was carried out as follows. That is, protoplasts were isolated from the leaves of *Arabidopsis thaliana,* which was transformed with pBI121-35Sp-UTR35:BiP:H5N1(HA):CBD:HDEL:NOS, and then lysed in W6 buffer (154 mM NaCl, 125 mM CaCl₂, 2.5 mM Maltose, 5 mM KCl, 10 mM HEPES, pH7.2). 300 µL of the transformed protoplasts lysed in the W6 buffer were placed on a slide coated with poly-L-lysine and 3% paraformaldehyde was treated thereto for about 1 hr. The transformed protoplasts was fixed and then washed with TWS buffer (10 mM Tris-HCl pH 7.4, 0.9% NaCl, 0.25% gelatin, 0.02% SDS, 0.1% Triton X-100). Polyclonal Anti-CBD (Rabbit) antibody as the primary antibody was allowed to react therewith and then the slide was washed again with TSW buffer (10 mM Tris-HCl pH7.4, 0.9% NaCl, 0.25% gelatin, 0.02% SDS, 0.1% Triton X-100). Then, rabbit-FITC (green fluorescence) as the secondary antibody was allowed to react and observation was carried out with a fluorescent microscope.

As shown in FIG. 4, the green fluorescent pattern was confirmed to be a typical network structure of the endoplasmic reticulum. Thus, it was found that the HA protein expressed from the transgenic plant prepared according to the present invention moved precisely to the endoplasmic reticulum (ER).

**Example 4**

**Examination of the glycosylation of the H5N1 hemagglutinin protein expressed from the transgenic plant**

Generally, the surface proteins of virus are glycosylated and such glycosylation induces antibody response and helps antibody formation. As described above, in order to induce more glycosylation of the H5N1 antigenic protein hemagglutinin, the present inventors transformed *Arabidopsis thaliana* with the recombinant vector enabling the transportation of a heterologous protein to the endoplasmic reticulum, where glycosylation occurs actively, and the retention of the heterologous proteins expressed in the endoplasmic reticulum for a longer time. The endoglycosidase H method was used to examine whether H5N1 hemagglutinin expressed in *Arabidopsis thaliana,* which was transformed by the method of the present invention, is glycosylated or not. Endoglycosidase H is an enzyme which cleaves selectively asparagines-linked oligosaccharides and the endoglycosidase H method has been used in the art to examine whether a protein is glycosylated or not. The more specific examination of protein glycosylation using the enzyme was as follows. Leaves of the transgenic *Arabidopsis thaliana* according to the present invention were collected and 100 µL of 1X homogenization buffer (25 mM Hepes pH 7.5, 1 mM DTT, 1 mM MgCl₂, 250 mM Sucrose) and a proteinase inhibitor were added thereto and proteins were extracted from the transgenic *Arabidopsis thaliana.* 100 µL of 4X denaturation buffer (2% SDS, 4% b-MeOH) was added to the protein extract and the protein extract was boiled at 100°C for 15 min and centrifuged at 12,000 rpm for 5 min and 200 µL of the supernatant was obtained. Then, 200 µL of 2X G5 reaction buffer (100 mM sodium citrate, pH 5.5) was added to the supernatant and 200 µL was taken as sample before enzyme treatment. To the remaining 200 µL, 1 µL of endoglycosidase H was added and treated at 37°C for 1 hr. Then, said samples were subjected to SDS-PAGE electrophoresis and a conventional Western blot analysis using polyclonal Anti-CBD (Rabbit) antibody was carried out to examine whether the H5N1 hemagglutinin was glycosylated or not.

As shown in FIG. 5, it was found that protein size in the sample treated with endoglycosidase H decreased as endoglycosidase H cleaved oligosaccharides more than in the sample which was not treated with endoglycosidase H. From the result, it was found that the HPAI (H5N1) type A/Hong Kong/213/03 HA protein expressed in the transgenic plant by the method of the present invention was normally glycosylated in the plant and suitable for the induction of antibody response to protein.

**Example 5**

**Separation and purification of H5N1 hemagglutinin protein from the transgenic plant**

The HPAI (H5N1) type A/Hong Kong/213/03 HA protein, the antigen of H5N1 virus, which causes avian influenza, was isolated and purified from the transgenic plant prepared in Example 2 according to the present invention as follows. About 70 to 80 seeds of the transgenic *Arabidopsis thaliana* were sowed in a 150mm MAXi plate and then cultivated in a culture room having the environmental condition of 16hr/8hr (light/dark) at 23°C for about 3 weeks. At this time, a culture medium containing 2% sucrose, B5 mixture, 0.5% MES pH 5.75, and 8% agar was included in the plate. Then, the cultivated *Arabidopsis thaliana* was placed in liquid nitrogen, and ground very finely. The extraction buffer (10 mM Hepes, 10 mM NaCl, 3 mM MgCl₂, 5 mM EGTA, 5 mM EDTA, 5 mM DTT, 0.2% Triton X-100) was added to the ground *Arabidopsis thaliana.* After centrifugation at 10,000 rpm for 10 min, only the supernatant was collected and loaded on a column of about 3 cm prepared with cellulose (Sigma type 20). When the loaded solution came down all the column, the column was washed with Washing buffer 1 (10 mM Hepes, 10 mM NaCl, 3 mM MgCl₂, 5 mM EGTA, 5 mM EDTA, 5 mM DTT, 0.2% Triton X-100) and washed again with Washing buffer 2 (10mM Hepes, 10 mM NaCl, 3 mM MgCl₂, 5 mM EGTA, 5 mM EDTA, 5 mM DTT). Then, the cellulose-bound BiP:H5N1(HA):CBD:HDEL fusion protein was purified with an elution buffer (1% cellobiose, 10 mM Hepes, 10 mM NaCl, 3 mM MgCl₂, 5 mM EGTA, 5 mM EDTA). The purified fusion protein was subjected to SDS-PAGE electrophoresis and stained with Coomassie to determine the amount of the purified protein.

In addition, in order to determine how many H5N1 virus HA proteins were produced from the transgenic *Arabidopsis thaliana* of the present invention, 100 ng of the isolated HA proteins from the isolated and purified H5N1 virus along with 50 µg of total water-soluble protein extracted from the transgenic *Arabidopsis thaliana* were subjected to SDS-PAGE electrophoresis and Western blot analysis using the CBD antibody was carried out. Then, the signal intensity was compared using a multigauge program and quantitative analysis was carried out.

As shown in FIG. 6a, the BiP:H5N1(HA):CBD:HDEL fusion protein was isolated and purified with very high purity from the transgenic *Arabidopsis thaliana* according to the present invention. As a result of the quantitative analysis of the protein, about 1 mg of protein could be obtained per one 150mm MAXi plate in which *Arabidopsis thaliana* were cultivated. As shown in FIG. 6b, the H5N1 virus HA protein expressed in the transgenic *Arabidopsis thaliana* was about 5% of total water-soluble proteins extracted from *Arabidopsis thaliana* and this figure is very excellent in comparison with the productivity of useful protein from conventional transgenic plants.

From the above result, it was found that the transgenic plant transformed with the vector for plant transformation according to the present invention could express and produce H5N1 virus HA proteins with very high efficiency.

**Example 6**

**Analysis of antibody formation response of H5N1 hemagglutinin antigen produced according to the present invention**

For hemagglutinin (HA) protein on the surface of HPAI (H5N1) type A/Hong Kong/213/03 virus to act as an immunizing antigen, a proper glycosylation is important. However, specific mechanism of glycosylation has delicate differences between plants and animals. In order to examine whether the hemagglutinin which was produced and glycosylated in plants has immunogenicity like the glycosylated protein produced in animal cells, antibody formation response was investigated using a mouse model. First, a Balb/c mouse which is widely used in influenza virus tests was used as the mouse model and the hemagglutinin protein obtained from the transgenic plant of the present method was used as a vaccine for antibody formation. The hemagglutinin protein obtained from the transgenic plant of the present method was diluted with PBS buffer to 10 µg/mL (H5N1 HA). For the control group, TIV (trivalent influenza vaccine, H1N1 + H3N2 + B type HA protein), which was not related with avian influenza, was diluted with PBS buffer to 30 µg/mL (TIV HA). Then, 50 µL of each vaccine protein per leg, a total of 100 µL of each vaccine protein was injected intramuscularly into both legs of the mouse twice, on 0 week and 3 week. Since three hemagglutinin proteins were mixed in TIV, the administration amount of TIV was three times greater than that of the H5N1 hemagglutinin protein. After each vaccine administration, analysis of antibody formation was carried out by collecting blood from suborbital veins using heparinized tubes at two weeks after the last administration, centrifuging the blood at 13,000 rpm for 10 min, and analyzing the serum in the supernatant. That is, the antigen-specific antibody response was investigated with the separated serum using ELISA method. As a target antigen for ELISA method, the hemagglutinin protein produced by the method of the present invention was used. The hemagglutinin protein was diluted with PBS buffer to 1.5 µg/mL and added 50 µL to each well in a 96-well absorption plate. The plate was allowed to stand still at 4°C overnight. Then, the plate was washed twice with 200 µL of PBST solution (PBS buffer solution containing 0.05% Tween-20) and 200 µL of a binding buffer (PBST solution containing 5% skim milk) was added to each well for 1 hr to block the plate. Then, the plate was washed twice with 200 µL of PBST solution. 50 µL of the serum diluted with the binding buffer at the ratio of 1:50 was added to each well and allowed to react at 37°C for 2 hr. Then, the plate was washed 5 times with 200 µL of PBST solution and anti-total IgG, IgG1, IgG2a antibodies which were coupled to HRP (horse radish peroxidase) were diluted with the binding buffer at the ratio of 1:3000 and added in an amount of 50 µL to each well and allowed to react at room temperature for 1.5 hr. After the reaction, the plate was washed 7 times with 200 µL of PBST solution. 50 µL of TMB substrate was added to each well to cause color development. When a proper color was developed, the color development reaction was stopped by adding 50 µL of 2N H₂SO₄ solution to each well. The optical density was determined at wavelength of 450nm using ELISA plate reader and the antibody response was measured.

As shown in FIG. 7, it was found that the control group administered with TIV could not induce H5N1-specific antibody response; however, the Balb/c mice administered with H5N1 had an immune response and induced the antigen-specific antibody response. In addition, this antigen-specific antibody response increased significantly through boost immunization.

Thus, from the result, the H5N1 hemagglutinin protein obtained by the method of the present invention had different form of glycosylation from the hemagglutinin protein obtained from the animal cells, but it was found that, it was immunogenic like the hemagglutinin protein obtained from the animal cells.

**Example 7**

**Measurement of the defensive effect of H5N1 hemagglutinin antigen produced according to the present invention against avian influenza virus**

From Example 6, it was confirmed that the H5N1 hemagglutinin protein produced according to the present invention could induce the antibody *in vivo,* and therefore, it could be found that the H5N1 hemagglutinin protein of the present invention had the defensive effect on the H5N1 avian influenza virus. Furthermore, the present inventors measured the defensive effect of the H5N1 hemagglutinin protein on the H5N2 avian influenza virus in order to investigate whether the H5N1 hemagglutinin protein exhibits the defensive effect actually on other kinds of avian influenza viruses. That is, mice to which the H5N1 hemagglutinin protein produced according to the present invention or TIV was inoculated were infected with 5LD₅₀ (LD₅₀: the dose to induce the fatality of 50% when infected) of H5N2 virus sufficient to induce the fatality rate of 100% of a nonimmunized mice population and changes in body weight of the mice and the survival rate after the infection were measured.

As shown in FIG. 8 and FIG. 9, in the control group to which TIV was administered, the body weight reduction, a typical symptom after H5N2 virus infection, was observed and most mice were dead between 6th day and 8th day after infection. On one hand, in the experimental group to which the H5N1 hemagglutinin protein derived from the plant, produced according to the present invention was administered, the body weight reduction was observed in all mice after the H5N2 virus infection, like the control group. However, the body weight of most mice gradually increased from about 10th day and was recovered to 90% or more of the initial body weights at about 15th day.

The survival rate of the mice in the TIV administration group was 0% (0/6), that is, all mice were dead by 15th day. However, the survival rate of the plant-derived H5N1 hemagglutinin administration group was 67% (4/6).

Considering that even if the subtype of influenza virus is the same, when the strain is different, defensive immunization may be difficult, through the fact that administration of H5N1 hemagglutinin according to the present invention induces effective defensive immunization also on the different subtype of virus, H5N2 avian influenza virus, from the above result, it could be found that the H5N1 hemagglutinin according to the present invention had an excellent defensive effect on H5N2 as well as H5N1. Furthermore, it could be confirmed that a new vaccine enough to substitute conventional vaccines against H5N1 and H5N2 could be developed. As the present inventors discovered a new virus vaccine against H5N1 virus through the above result, it can be found that the protein vaccine can be used for a vaccine against H5N1 virus for not only animals infected with H5N1 virus but also all animals including birds and humans, based on the report that H5N1 virus has recently infected humans.

**Example 8**

**Examination of a use of the H5N1 hemagglutinin according to the present invention for a marker vaccine through the CBD antibody test**

Based on the idea that in addition to the hemagglutinin gene, the CBD gene was also used for the effective separation and purification of proteins in the vector used in the present invention for plant transformation, as the H5N1 hemagglutinin protein expressed by the method according to the present invention was fused to the CBD protein, the present inventors examined whether the antibody against the CBD protein was also formed or not. That is, the H5N1 hemagglutinin protein produced from the transgenic *Arabidopsis thaliana* according to the present invention, i.e., the H5N1 hemagglutinin protein-CBD fused protein, was injected as an antigen into a mouse. Then, Western blot analysis was carried out using serum obtained from the mouse in order to examine whether the serum of the mouse immunized with the vaccine of the present invention contains an antibody which can recognize the CBD protein or not. The Western blot analysis was carried out equally by conventional Western blot analysis. Serum obtained from a rabbit immunized with the CBD protein only was used as a comparative antibody in the Western blot analysis.

As shown in FIG. 10, serum of the mouse immunized with the vaccine of the present invention, i.e., the H5N1 hemagglutinin protein-CBD fused protein, recognized not only the vaccine protein (H5N1(HA):CBD) which was used for the immunization, but also the CBD band from transgenic plant producing unspecific CBD (GFP:CBD, TPA:CBD). Thus, it was found that the CBD which was coupled to hemagglutinin also induced the antibody response in the mouse. When the experiment in which the CBD antibody was used with the same sample was performed, the same band with the CBD antibody was detected from the immunized mouse. Thus, it was found that the vaccine used in the present invention produced sufficient antibodies to protect mice from virus and at the same time, the vaccine also produced the antibody against CBD.

Accordingly, from the CBD antibody test result, the present inventors could predict that if both the antibody against hemagglutinin and the antibody against CBD are detected in the serum test for the diagnosis of avian influenza, the antibody is formed as a result of the vaccine inoculation, and if the CBD antibody is not detected, but, only the antibody against hemagglutinin is detected, the antibody is formed by the virus infection.

Therefore, the detection method using the CBD antibody according to the present invention can determine whether the detected antibody is formed either by vaccine inoculation or viral infection through analysis of the antibody detected from the clinical specimen. Thus, the antigenic protein which is produced from the transgenic plant and provided by the present invention, i.e., the H5N1 hemagglutinin protein-CBD fused protein, can be used for a marker vaccine. With this, the present inventors can expect that unnecessary destruction of poultry can be prevented and economical losses can be reduced and the quick treatment for early human infection can be possible.

**Example 9**

**Analysis of antibody formation of the H5N1 hemagglutinin antigen produced according to the present invention in a bird model**

From the fact that the H5N1 hemagglutinin protein produced according to the present invention can induce the antibody response through the above mouse experiment in Example 6, it could be found that the H5N1 hemagglutinin protein produced according to the present invention can be used for the development of the vaccine for mammals, including humans. Furthermore, in order to examine whether the H5N1 hemagglutinin protein produced according to the present invention can be used for the development of a vaccine for poultry or not, the present inventors investigated whether the H5N1 hemagglutinin protein induces the antibody response in chickens or not through the following experiment. Chickens were used for the poultry and White leghorn breed chickens were used regardless of sex. Two weeks after hatch, pre-serum samples were collected from chickens and 5 µg, 25 µg, and 50 µg of the hemagglutinin protein obtained from the plant according to the present invention was administered three times at two-week intervals. For the control group, instead of the hemagglutinin protein, PBS buffer was administered three times at two-week intervals. At two weeks after the last administration, blood samples were collected using heparinized tubes and centrifuged at 13,000 rpm for 10 min and the supernatant, serum was separated. The antigen-antibody response was carried out with the separated serum using ELISA method that is widely used in the art. As a target antigen for ELISA method, the hemagglutinin protein produced by the method of the present invention was used. The hemagglutinin protein was diluted with PBS buffer to 1.5 µg/mL and added 50 µL to each well in a 96-well absorption plate. The plate was allowed to stand still at 4°C overnight. The next day, the plate was washed twice with 200 µL of PBST solution (PBS buffer solution containing 0.05% Tween-20) and 200 µL of a binding buffer (PBST solution containing 5% skim milk) was added to each well and the plate was blocked for 1 hr. Then, the plate was washed twice with 200 µL of PBST solution. 50 µL of the serum diluted with the binding buffer at the ratio of 1:50 was added to each well and allowed to react at 37°C for 2 hr. Then, the plate was washed 5 times with 200 µL of PBST solution and anti-total IgG (chicken) antibody which was coupled to HRP (horse radish peroxidase) was diluted with the binding buffer at the ratio of 1:3000 and added in an amount of 50 µL to each sample and allowed to react at room temperature for 1.5 hr. After the reaction, the plate was washed 13 times with 200 µL of PBST solution. 50 µL of TMB substrate was added to each well to cause color development. The color development reaction was stopped by adding 50 µL of 2N H₂SO₄ solution to each well. The optical density was determined at wavelength of 450nm using ELISA plate reader and the antibody response was measured.

As shown in FIG. 11, it was found that chickens administered with PBS buffer for the control group could not induce H5N1-specific antibody response; however, White leghorn chickens administered with the H5N1 hemagglutinin protein produced according to the present invention was immunogenic regardless of sex and induced the antigen-specific antibody response. In addition, this antigen-specific antibody response increased significantly through boost immunization.

From the result, the present inventors found that the H5N1 hemagglutinin protein obtained by the method of the present invention had a different form of glycosylation from the hemagglutinin protein obtained from the animal cells. However, the H5N1 hemagglutinin protein of the present invention obtained from plants, had the same immunogenicity to the hemagglutinin protein obtained from the animal cells from the experiment result using the chicken model. Furthermore, the present inventors found that the H5N1 hemagglutinin protein of the present invention can be used for the preparation of a vaccine for poultry including chickens, as well as for mammals including humans.

Hitherto, the present invention is looked into around the preferred embodiments. Those skilled in the art will understand that the present invention may be embodied in modified forms without departing from the intrinsic characteristic of the present invention. Therefore, the disclosed embodiments should be considered from illustrative perspective, but not from limitative perspective. The scope of the present invention is represented in the accompanying claims rather than the above description and it should be understood that all the differences within the equal scope are included in the present invention.

## Claims

1. A method of preparing a transgenic plant producing antigenic hemagglutinin (HA) protein of H5N1 virus, the method comprising introducing a vector for plant transformation into a plant, wherein the vector comprises a gene construct consisting of (i) a DNA fragment having nucleotide sequence selected from the group consisting of SEQ ID NOs: 1 to 12; (ii) BiP (chaperone binding protein) gene having nucleotide sequence of SEQ ID NO: 13; (iii) a polynucleotide encoding hemagglutinin (HA) protein of H5N1 virus, having nucleotide sequenceof SEQ ID NO: 14, (iv) a polynucleotide encoding a cellulose-binding domain (CBD), having nucleotide sequence of SEQ ID NO: 15; and (v) a polynucleotide encoding HDEL (His-Asp-Glu-Leu) peptide serially, wherein the gene construct is operably linked to a promoter.

2. The method as set forth in claim 1, wherein the method of introducing the vector for plant transformation into a plant may be any one selected from the group consisting of *Agrobacterium* sp.-mediated transformation, particle gun bombardment, silicon carbide whiskers, sonication, electroporation, and PEG (polyethylene glycol) precipitation.

3. The method as set forth in claim 1, wherein the plant may be dicotyledonous plants selected from the group consisting of *Arabidopsis thaliana,* soybeans, tobaccos, eggplants, red peppers, potatoes, tomatoes, Chinese cabbages, Chinese radishes, cabbages, lettuces, peaches, pears, strawberries, watermelons, muskmelons, cucumbers, carrots and salaries; or monocotyledonous plants selected from the group consisting of rice, barley, wheat, rye, corn, sugarcane, oats and onions.

4. A transgenic plant producing an antigenic hemagglutinin (HA) protein of H5N1 virus, prepared by the method of any one of claims 1 to 3.

5. A method of producing an antigenic hemagglutinin (HA) protein of H5N1 virus, from the transgenic plant of claim 4, which comprises cultivating the transgenic plant and isolating and purifying the HA protein from the transgenic plant.

6. The method as set forth in claim 5, wherein the antigenic hemagglutinin (HA) of H5N1 is a fused form with a cellulose-binding domain (CBD).

7. A vaccine protein against H5N1 virus produced by the method of claim 5 or claim 6.

8. The vaccine protein as set forth in claim 7, wherein the protein is a cellulose-binding domain (CBD)-fused H5N1 hemagglutinin (HA) protein.

9. A vaccine composition against H5N1 virus, which comprises the transgenic plant of claim 5 or claim 6, an H5N1 hemagglutinin (HA) protein produced from the transgenic plant, or protein extracts of the transgenic plant.

10. A method of diagnosing whether an antibody in a clinical specimen is formed by the infection of H5N1 virus or vaccine administration, which comprises the steps of: (a) collecting blood from the clinical specimen; (b) separating serum from the collected blood; and (c) adding an antibody against hemagglutinin (HA) and an antibody against cellulose-binding domain (CBD) to the separated serum to react.

11. The method as set forth in claim 10, wherein it is determined that the antibody in the clinical specimen is formed by the administration of the vaccine composition of claim 9 if both the antibody against hemagglutinin (HA) and the antibody against cellulose-binding domain (CBD) are detected in the reaction of the step (c), and the antibody in the clinical specimen is formed by the infection of H5N1 virus if only the antibody against hemagglutinin (HA) is detected.
